# EUROPEAN PATENT APPLICATION

(11) **EP 2 979 642 A1**
(43) Date of publication of application: **03.02.2016**
(21) Application number: 14774646.5
(22) Date of filing: 20.03.2014
(51) Int. Cl.: A61B 8/00

(54) **IMAGE ALIGNMENT DISPLAY METHOD AND ULTRASONIC DIAGNOSTIC DEVICE**

(30) Priority: 29.03.2013 JP 2013074572
(71) Applicant: Hitachi Aloka Medical, Ltd., Tokyo 181-8622 (JP)
(72) Inventor: HIRAI, Takanori, Mitaka-shi Tokyo 181-8622 (JP)
(74) Representative: Strehl Schübel-Hopf & Partner
(86) International application number: PCT/JP2014/057807
(87) International publication number: WO 2014/156973

(57) **Abstract**

Problem to be Solved

To make it possible to simplify an image alignment process and shorten time therefor.

Solution

Characteristics are: performing a process for alignment between an ultrasound image (a US image) generated on the basis of a reflected echo signal of a cross-section plane of a diagnosing object received with an ultrasound probe and a reference image (an R image) obtained by another image diagnostic apparatus to display the images on a display screen 22 of an image displaying section; storing a plurality of results of the alignment process together with alignment data and capture images 20; displaying the stored capture images 20 on the display screen 22 as a list; and, when one of the displayed capture images 20 is selected, performing the alignment process by the alignment data corresponding to a capture image 20 to which a selection mark 21 is attached.

## Description

### Technical Field

The present invention relates to an image alignment display method and an ultrasonic diagnostic apparatus and relates to an image alignment display method for aligning the position of a diagnostic image obtained by a different image diagnostic apparatus to display the diagnostic image on a display screen, and an ultrasonic diagnostic apparatus.

### Background Art

In the image diagnosis field, it is performed to display an ultrasound image obtained by an ultrasonic diagnostic apparatus in real time and a reference image obtained an image of the same region of a diagnosing object by another image diagnostic apparatus being compared or being overlapped. For example, a method for performing alignment between an ultrasound image and a reference image is described in Patent Literature 1 and Patent Literature 2. Especially, because, even if positions of an ultrasound image and a reference image are aligned, the alignment is displaced when a diagnosing object moves due to a body motion, breathing or the like, it is proposed to perform alignment between the images again.

### Citation List

### Patent Literature

Patent Literature 1: JP-A-2008-246264
Patent Literature 2: JP-A-2009-90120

### Summary of Invention

### Technical Problem

However, there is often a case where, even if image alignment at a certain diagnosis region is appropriate, the alignment is displaced when a diagnosis region is moved to a different position. In that case, image alignment is to be performed each time the diagnosis region is moved, and there is room for improvement of Patent Literatures 1 and 2 in solving the complicatedness of the alignment process and shortening time.

A problem to be solved by the present invention is to make it possible to simplify an image alignment process and shorten processing time therefor.

### Solution to Problem

In order to solve the above problem, an image alignment display method of the present invention is characterized in: performing a process for alignment between an ultrasound image generated on the basis of a reflected echo signal of a cross-section plane of a diagnosing object received with an ultrasound probe and a reference image obtained by another image diagnostic apparatus to display the images on an image displaying section; storing a plurality of alignment results of the alignment process together with alignment data and correspondence-for-alignment images; displaying the stored correspondence-for-alignment images on the image displaying section as a list; and, when one of the displayed correspondence-for-alignment images is selected, performing the alignment process by the alignment data corresponding to the selected correspondence-for-alignment image.

### Advantageous Effects of Invention

According to the present invention, it is possible to simplify an image alignment process and shorten processing time therefor.

### Brief Description of Drawings

[Figure 1] Figure 1 is a block configuration diagram of an embodiment to which an image alignment display method of the present invention is applied.
[Figure 2] Figure 2 is a flowchart of an image alignment display method of an example 1 of the present invention.
[Figure 3] Figure 3 is a diagram illustrating an operation of the example 1.
[Figure 4] Figure 4 is a flowchart of an image alignment display method of an example 2 of the present invention.
[Figure 5] Figure 5 is a diagram illustrating an operation of the example 2.
[Figure 6] Figure 6 is a flowchart of an image alignment display method of an example 3 of the present invention.
[Figure 7] Figure 7 is a diagram illustrating an operation of the example 3.
[Figure 8] Figure 8 is a flowchart of an image alignment display method of an example 4 of the present invention.
[Figure 9] Figure 9 is a diagram illustrating an operation of the example 4.
[Figure 10] Figure 10 is a flowchart of an image alignment display method of an example 5 of the present invention.
[Figure 11] Figure 11 is a diagram illustrating an operation of the example 5.

### Description of Embodiments

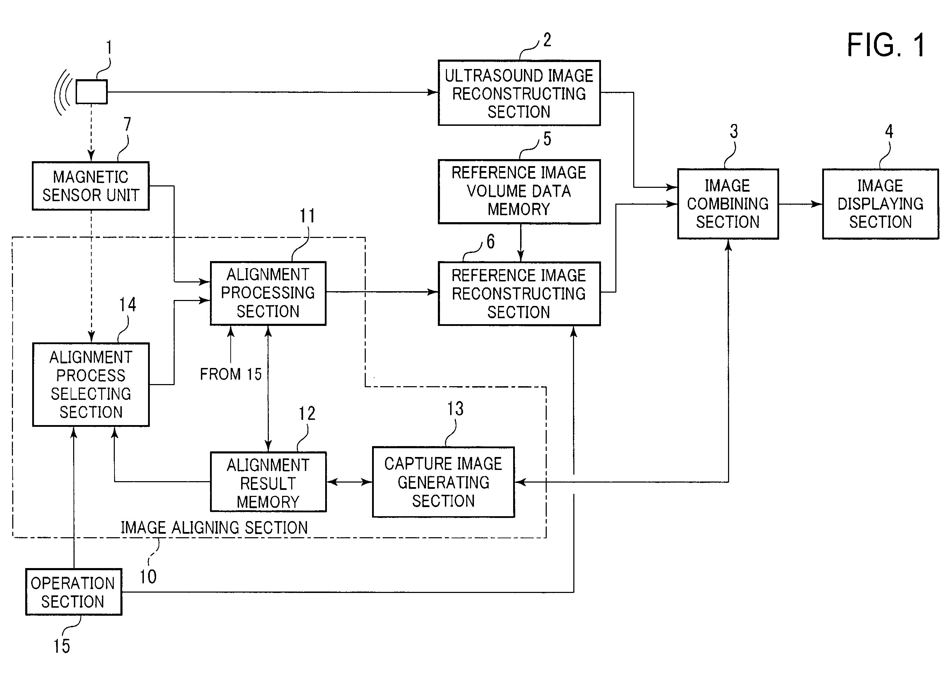

Description will be made below on the basis of an embodiment and examples of an image alignment display method of the present invention and an ultrasonic diagnostic apparatus to which the method is applied. Figure 1 is a block configuration diagram of one embodiment of the ultrasonic diagnostic apparatus of the present invention. As shown, there are provided an ultrasound image reconstructing section 2 which generates an ultrasound image in real time on the basis of a reflected echo signal of a cross-section plane of a diagnosing object received by an ultrasound probe 1, an image combining section 3 which combines the ultrasound image generated by the ultrasound image reconstructing section 2 with another image, and an image displaying section 4 which displays the combined image. Further, there are provided a volume data memory 5 in which volume data of a reference image obtained by another image diagnostic apparatus is to be stored, and a reference image reconstructing section 6 which reads out reference image data corresponding to an ultrasound image from the volume data memory 5 to generate a reference image. Here, various modalities, such as an X-ray diagnostic apparatus, an MRI diagnostic apparatus, a CT diagnostic apparatus, an ultrasonic diagnostic apparatus or a PET diagnostic apparatus, can be applied to that other image diagnostic apparatus. The volume data is constructed with a plurality of cross-section region image data obtained at a plurality of parallel cross-section planes for a diagnosing object's diagnosis region. This volume data is stored into the volume data memory 5 from that other image diagnostic apparatus not shown, via a signal transmission line or a storage medium.

A magnetic sensor unit 7 is configured with a magnetic field generating device which causes a magnetic field to occur in a space which includes a diagnosing object to be image-diagnosed by the ultrasonic diagnostic apparatus of the present embodiment, and a magnetic sensor attached to the ultrasound probe 1. The magnetic sensor unit 7 is adapted to detect a position and inclination angle (hereinafter referred to simply as an angle) of the ultrasound probe 1 and input them to an alignment processing section 11 of an image aligning section 10.

The alignment processing section 11 is adapted to calculate a position and inclination angle (hereinafter referred to simply as an angle) of a cross-section plane (a scan plane or a scanning plane) which the ultrasound probe 1 forms inside a diagnosing object on the basis of the inputted position and angle of the ultrasound probe 1. Coordinates data of a real-time ultrasound image displayed on the image displaying section 4 is calculated on the basis of the calculated position and angle of the cross-section plane. Next, coordinates data of a reference image corresponding to the ultrasound image is calculated with the use of a coordinate conversion formula for image alignment which is set in advance. That is, as is known, the coordinate system of the ultrasonic diagnostic apparatus and the coordinate system of another image diagnostic apparatus which has obtained the reference image are set in association with each other with a diagnosing object as a base. In order to associate the coordinate systems of the two image diagnostic apparatuses, a coordinate conversion formula for bidirectionally converting two pieces of coordinate data to be associated with is set.

The reference image reconstructing section 6 reads out reference image data corresponding to the coordinate data of the reference image determined by the alignment diagnostic section 11 from the volume data memory 5, generates the reference image and outputs the reference image to the image combining section 3. The image combining section 3 combines the ultrasound image outputted from the ultrasound image reconstructing section 2 and the reference image outputted from the reference image reconstructing section 6 and causes the combined image to be displayed on the image displaying section 4. In the present embodiment, image combination includes a case where both images are combined by being overlapped with each other with a set ratio, in addition to an example in which both images are displayed being arranged side by side.

Next, a configuration related to characteristic sections of the present embodiment will be described. The image aligning section 10 is configured being provided with the alignment processing section 11, an alignment result memory 12, a capture image generating section 13 and an alignment process selecting section 14. The alignment processing section 11 adjusts a parameter of the coordinate conversion formula in accordance with a positional displacement adjustment instruction which an operator inputs from an operation section 15, if there is positional displacement between the ultrasound image and the reference image associated on the basis of the coordinate conversion formula set initially as described before. For example, the operator freezes the reference image, changes the position and angle of the ultrasound probe 1, causes a real-time ultrasound image corresponding to the reference image to be displayed on the image displaying section and inputs an alignment termination instruction from the operation section 15. Thereby, the alignment processing section 11 performs adjustment of the parameter of the coordinate conversion formula and the like, and stores alignment adjustment data therefor into the alignment result memory 12 as alignment data, together with other related alignment data. Here, items of the alignment data stored into the alignment result memory 12 includes various conditions involved in alignment, and the conditions can be appropriately set as necessary.

For example, the alignment adjustment data outputted from the alignment processing section 11, such as the alignment adjustment data, the kind (modality) of the image diagnostic apparatus which has obtained the reference image, an identification number of reference image volume data, and a position and angle of the ultrasound probe (cross-section plane) detected by the magnetic sensor, can be stored into the alignment result memory 12. Furthermore, corresponding image data of an aligned ultrasound image and a reference image (hereinafter referred to as capture image data) is stored into the alignment result memory 12 in association with the alignment adjustment data. As for the capture image data, the capture image generating section 13 is adapted to capture image data corresponding to the ultrasound image and the reference image from the image combining section 3 and store the capture image data into the alignment result memory 12, at a timing of the alignment termination instruction being inputted from the operation section 15.

Further, the capture image generating section 13 is adapted to generate capture images, which is an alignment comparison image, on the basis of the captured capture image data and capture image data stored in the alignment result memory 12, and display the capture images as a list on the image displaying section 4 via the image combining section 3. As for the display format of the list, the list can be displayed on the image displaying section 4 with thumbnail images. In the case of displaying thumbnail images as a list, the image combining section 3 can arrange and display the thumbnail images together with an ultrasound image and a reference image at a lower part of the screen of the image displaying section 4. The list display, however, is not limited to thumbnail images. In short, any image format is possible if the format is in an aspect of making it possible to check a capture image and judge whether alignment is appropriate or not. The display position is not limited to the lower part of the image displaying section 4 but can be appropriately selected. Furthermore, the alignment adjustment data may be displayed on the image displaying section 4 together.

On the other hand, the alignment process selecting section 14 outputs an instruction to cause an alignment process to be performed, to the alignment processing section 11 in accordance with an instruction inputted from the operation section 15, that is, in accordance with alignment data corresponding to one capture image which the operator has selected from among the capture images displayed as a list on the image displaying section 4. In response thereto, the alignment processing section 11 reads out alignment adjustment data corresponding to the selected capture image from the alignment result memory 12 and outputs coordinate data of a reference image corresponding to a real-time ultrasound image to the reference image reconstructing section 6. Thereby, an alignment process in accordance with an alignment result of the capture image the operator has selected is performed.

A detailed configuration and an operation will be described about the image aligning section 10 of the ultrasonic diagnostic apparatus of the one embodiment configured as described above, by examples.

### Example 1

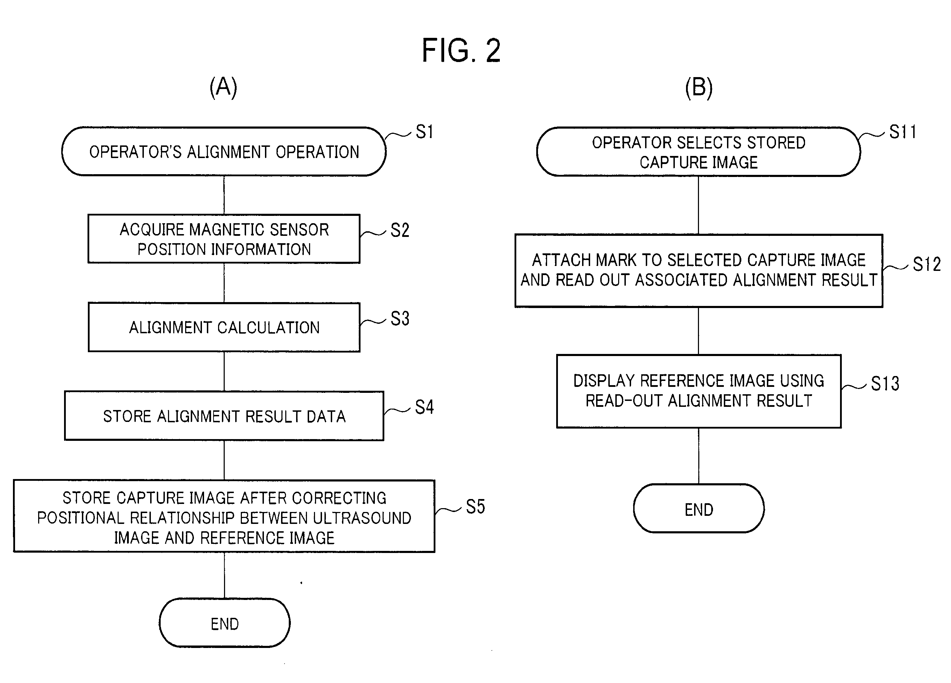

In Figure 2, a process procedure of the image aligning section 10 of an example 1 is shown as a flowchart. If the operator judges that a real-time ultrasound image and a real-time image displayed on the image displaying section 4 are displaced from each other, the operator adjusts the position and angle of the ultrasound probe 1 and performs alignment (S1) as shown in Figure 2(A). That is, the operator freezes the reference image, changes the position and angle of the ultrasound probe 1, and causes a real-time ultrasound image corresponding to the reference image to be displayed on the image displaying section. The alignment processing section 11 acquires position information (a position and an angle) of the magnetic sensor from the magnetic sensor unit 7 (S2). Next, the alignment processing section 11 calculates coordinate data of the real-time ultrasound image on the basis of the position information of the magnetic sensor, and executes an alignment calculation on the coordinate data for adjusting a parameter of a conversion matrix of the coordinate conversion formula so that the coordinate data of the reference image corresponds to the coordinate data (S3). Then, alignment data including parameter-adjusted data is stored into the alignment result memory 12 as alignment result data at the time of the operator's alignment termination instruction being inputted from the operation section 15 (S4). Furthermore, the capture image generating section 13 acquires capture image data, which is a correspondence image of the ultrasound image and the reference image after their positional relationship having been corrected, from the image combining section 3, stores the capture image data into the alignment result memory 12 (S5), and ends the image alignment process.

Though description has been made with the example in which the operator freezes a reference image, changes the position and angle of the ultrasound probe 1, and causes a real-time ultrasound image corresponding to the reference image to be displayed on the image displaying section, it is also possible to, on the contrary, freeze the ultrasound image, change coordinate data of the reference image to be aligned with the frozen ultrasound image.

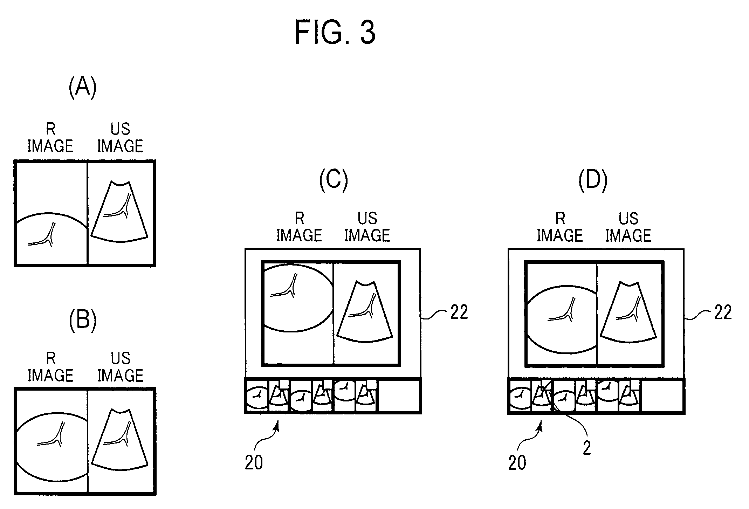

Generally, the ultrasound probe 1 may be moved to pick up a diagnosis region of a diagnosing object from a different position or angle. When the position and angle of the ultrasound probe 1 changes, however, it may happen that the correspondence relationship between an ultrasound image displayed in real time (a US image) and a reference image (an R image) is displaced as shown in Figure 3(A). Therefore, when the operator judges that the correspondence relationship between an ultrasound image and a reference image which are displayed on the image displaying section 4 is displaced, the operator executes the alignment process of Figure 2(A). Thereby, the R image corresponding to the US image is displayed as shown in Figure 3(B).

In this way, each time the alignment process is executed, alignment adjustment data and capture image data are stored into the alignment result memory 12. The capture image generating section 13 generates capture images on the basis of the capture image data stored in the alignment result memory 12 and displays the capture images as a list on the image displaying section 4 via the image combining section 3 as shown in Figure 3(C). This list display is displayed together with an ultrasound image and a reference image, being reduced to thumbnail images 20.

By the way, during the course of performing the image alignment process, the alignment process for determining an optimal relationship between an ultrasound image and a reference image is repeatedly performed while the position and angle of the ultrasound probe 1 is changed little by little. Such an alignment process requires complicated operations and also requires a lot of processing time. Therefore, in the present example, the complicated operations for the alignment process are avoided to simplify the alignment process and shorten processing time by utilizing past alignment results stored in the alignment result memory 12, as shown in Figure 2(B). That is, as shown in Figure 3(D), one thumbnail image which is in an appropriate correspondence relationship between an ultrasound image and a reference image is selected from among the plurality of thumbnail images 20 displayed as a list on the image displaying section 4 by the operator's judgment (S11). This selection is performed, for example, by attaching a mark 21 to the capture image 20 selected by the operation section 15.

In response thereto, the alignment process selecting section 14 reads out alignment adjustment data corresponding to the one capture image which the operator has selected, from the alignment result memory 12 in accordance with an instruction inputted from the operation section 15 (S12) and outputs the alignment adjustment data to the alignment processing section 11. The alignment processing section 11 determines coordinate data of a reference image corresponding to a real-time ultrasound image in accordance with the inputted alignment adjustment data. Then, the alignment processing section 11 outputs the determined coordinate data of the reference image to the reference image reconstructing section 6 to reconstruct the reference image corresponding to the selected capture image and display the reference image on the image displaying section 4 (S13).

According to the present example, when the operator judges that a result of alignment performed previously is appropriate in the course of performing the image alignment process, the operator can quickly restore the previous alignment result by selecting a capture image corresponding thereto. As a result, it is possible to avoid complicated operations for the alignment process to simplify the alignment process and shorten processing time.

In the present example, the example in which the alignment process is performed on the basis of alignment adjustment data corresponding to a capture image which the operator has selected. The alignment process selecting section 14, however, can select an alignment result stored in the alignment result memory 12 on the basis of at least one of detected values of the position and angle of the ultrasound probe 1. Further, the alignment processing section 11 can be formed being provided with a function of displaying the alignment adjustment data on the image displaying section 4.

### Example 2

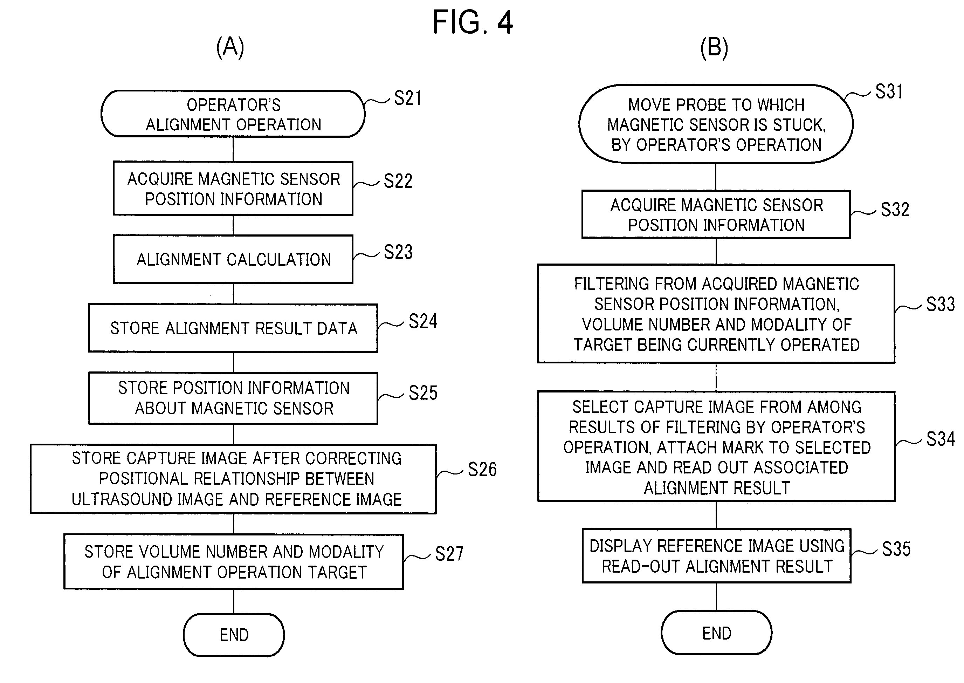

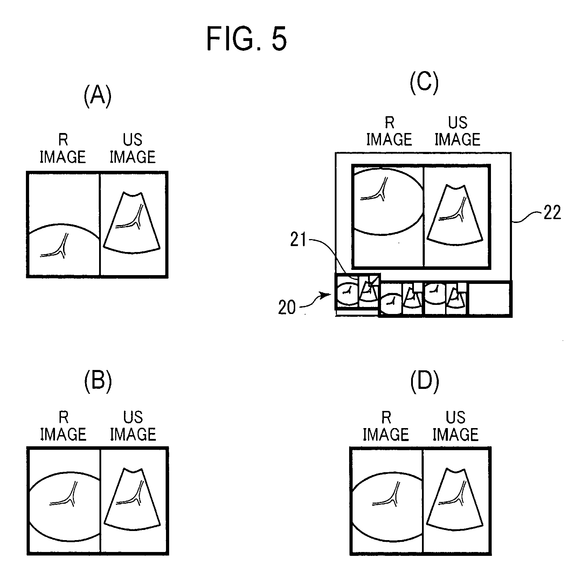

In Figure 4, a process procedure of the image aligning section 10 of an example 2 is shown as a flowchart. Figure 4(A) is a process performed when the operator judges that a real-time ultrasound image (a US image) and a reference image (an R image) displayed on the image displaying section 4 are displaced from each other as in Figure 5(A). Since steps S21 to S24, and S26 in Figure 4(A) correspond to steps S1 to S4, and S5 in Figure 2, respectively, detailed description thereof will be omitted. Further, Figure 5(B) shows the ultrasound image (the US image) and the reference image (the R image) which have been aligned according to the example 2.

Points in which the present example is different from the example 1 are that position information of the magnetic sensor is stored into the alignment result memory 12 (S25) and that an identification number of volume data of a reference image targeted by an alignment operation and the kind of modality which has obtained the reference image are stored into the alignment result memory 12 (S27), and then the image alignment process is ended.

Figure 4(B) is a process procedure in the case of repeatedly performing the image alignment process to determine an optimal relationship between an ultrasound image and a reference image while changing the position and angle of the ultrasound probe 1 little by little during the course of performing the image alignment process, and it is a process corresponding to Figure 2(B) of the example 1. It is the same as the example 1 that, at step S31, the ultrasound probe 1 to which the magnetic sensor is stuck is moved by the operator's operation. Next, the alignment processing section 11 acquires position information (a position and an angle) of the magnetic sensor (S32). Then, at step 33, filtering of alignment results stored in the alignment result memory 12 is performed on the basis of the position information of the magnetic sensor acquired at step S32, an identification number of volume data of a reference image being currently operated. That is, such an alignment result that has the same identification number of volume data and the same modality and that the position information of the magnetic sensor is within a predetermined permissible range is extracted. Next, at step S34, the mark 21 is attached to the capture image 20 selected by the operator from among the filtered alignment results, as shown in Figure 5(C). Then, an alignment result of the capture image to which the mark 21 is attached is read out from the alignment result memory 12 by the alignment process selecting section 14 and is outputted to the alignment processing section 11. The alignment processing section 11 determines coordinate data of a reference image corresponding to a real-time ultrasound image in accordance with inputted alignment data. Then, the alignment processing section 11 outputs the determined coordinate data of the reference image to the reference image reconstructing section 6 to reconstruct a reference image corresponding to the selected capture image and display the reference image on the image displaying section 4 as shown in Figure 5(D).

Since a configuration is made as above, filtering is performed with position information of the magnetic sensor, an identification number of volume data and a modality to display capture images of filtered alignment results as a list, at the time of utilizing a plurality of alignment results stored in the past, and, therefore, the alignment process by the operator becomes easier, according to the example 2. That is, the alignment process selecting section 14 of the present example extracts such alignment results stored in the alignment result memory 12 that the kind (modality) of another image diagnostic apparatus which has obtained a reference image and an identification number of volume data of the reference image correspond to display capture images as a list.

### Example 3

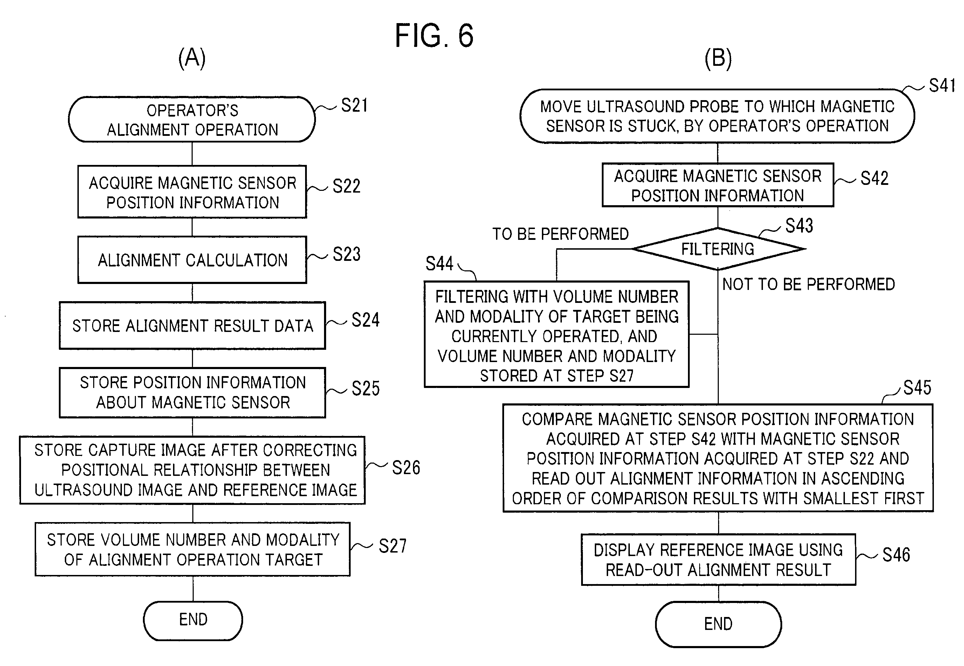

In Figure 6A, a process procedure of the image aligning section 10 of an example 3 is shown as a flowchart. Since a procedure related to an alignment process of the present example is the same as the example 2 as shown in Figure 6(A), the same reference numeral is given to each step, and description thereof will be omitted. Steps S41, S42 and S46 in Figure 6(B) correspond to the processes of S31, S32 and S35 in Figure 4 of the example 2, respectively. Points in which the present example is different from the example 2 exist in steps S43, S44 and S45 of the flowchart of Figure 6(B). That is, after acquiring position information of the magnetic sensor at step S42, the alignment processing section 11 judges whether or not to perform filtering for using alignment results stored in the alignment result memory 12 on the basis of an instruction inputted from the operation section 15 (S43). When filtering is to be performed, filtering is performed with the identification number and modality of volume data being currently operated, and the volume number and modality stored at step S27 (S44).

Then, no matter whether filtering is performed or not, the process proceeds to step S45, where the alignment result memory 12 is searched, the position information of the magnetic sensor acquired at step S42 and the position information of the magnetic sensor acquired at step S22 are compared, and alignment results are read out in ascending order of comparison results with the smallest first. That is, in the present example, the alignment process selecting section 14 compares at least one of detected values of the position and angle of the ultrasound probe 1 and detected values of the positions and angles of the ultrasound probe 1 of alignment results stored in the alignment result memory 12, and selects an alignment result corresponding to a detected value with a small difference. Then, the alignment processing section 11 determines coordinate data of a reference image corresponding to a real-time ultrasound image and outputs the coordinate data to the reference image reconstructing section 6, in accordance with the read-out alignment result, and displays the reference image reconstructed by the reference image reconstructing section 6 on the image displaying section 4.

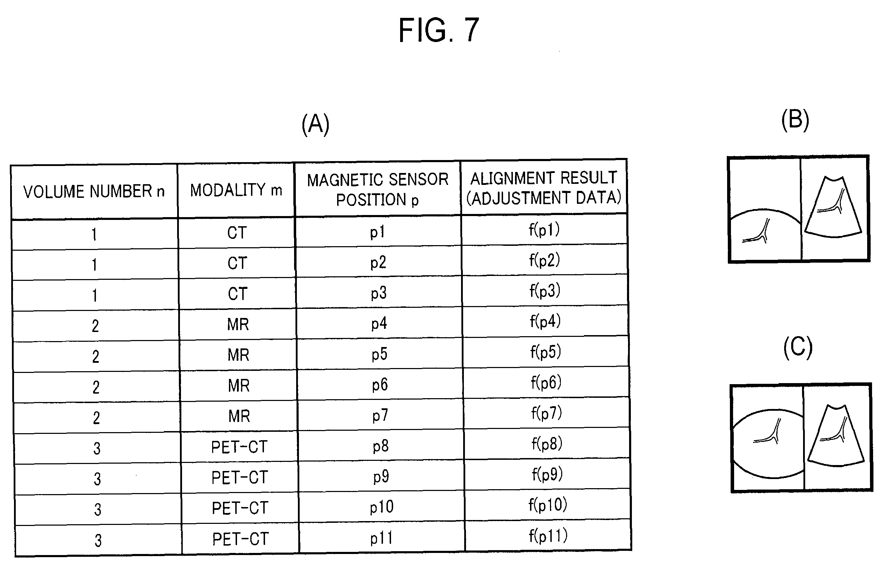

An example of a table of alignment result data, which is the alignment data stored in the alignment result memory 12 according to the example 3 is shown in Figure 7(A). As shown, volume data identification number n, modality m, magnetic sensor position pi and alignment adjustment data f(pi) are stored. Here, i is a natural number and given, for example, as a consecutive number. The magnetic sensor position pi acquired and stored at step S22 in Figure 6(A) is acquired a plurality of times as necessary. Then, in the comparison between a current magnetic sensor position pi' acquired at step S42 in Figure 6(B) and pi at step S45, comparison with all of p1 to p11 stored in the alignment result memory 12 is performed. When filtering is performed with the modality m, however, comparison with p1 to p3 is performed if the modality m is CT. Then, the alignment processing section 11 reads out alignment adjustment data f(pi) corresponding to pi which shows the smallest value as a result of the comparison, and executes the alignment process in accordance with this alignment adjustment data f(pi). As a result, positions of a US image and an R image displaced from each other as shown in Figure 7(B) are adjusted as shown in Figure 7(C).

### Example 4

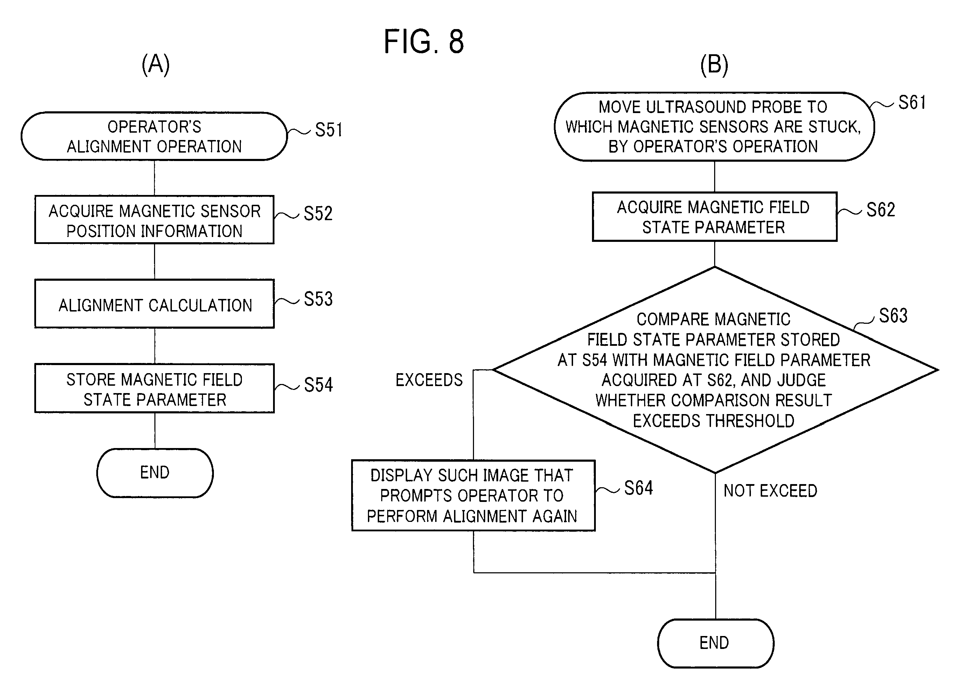

In Figure 8, a process procedure of the image aligning section 10 of an example 4 is shown as a flowchart. The present example is characterized in judging a magnetic field state of the magnetic sensor unit 7 and, if the magnetic field state is inappropriate, displaying a message as an image for causing a performed alignment process to be performed again on a GUI (graphic user interface) provided on the image displaying section 4 or the operation section 15. That is, as shown in Figure 8(A), steps S51 to S53 are the same processes as S1 to S3 of the example 1 of Figure 2. In the example 4, a magnetic field state parameter at the time of operating the ultrasound probe 1 to perform alignment is acquired at step S51, and the acquired magnetic field state parameter is stored into the alignment result memory 12

(S54). Here, as for the magnetic field state parameter, for example, a plurality of magnetic sensors are stuck to the ultrasound probe 1, and it is always continued to calculate a distance among the magnetic sensors. The distance is regarded as the magnetic field state parameter, and it is possible to, if the magnetic field state parameter decreases or increases, judge that the magnetic field is disordered. Then, the alignment processing section 11 stores the inputted or determined magnetic field state parameter into the alignment result memory 12.

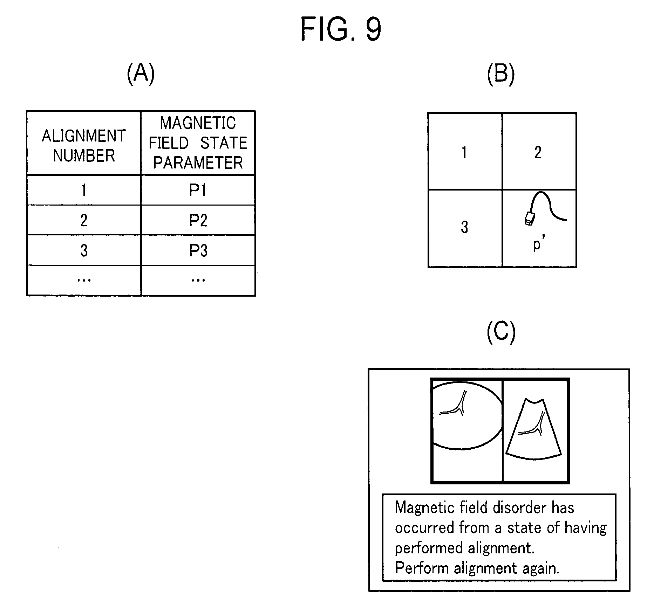

Next, at the time of performing the alignment process, the alignment processing section 11 executes the process like the flowchart in Figure 8(B). That is, at step S61, the operator causes the ultrasound probe 1 to which the magnetic sensors are stuck to move and aligns an ultrasound image with a frozen reference image similarly to the example 1. Next, the alignment processing section 11 acquires a magnetic field state parameter (S62). Then, the alignment processing section 11 compares one or a plurality of magnetic field state parameters stored in the alignment result memory 12 with the magnetic field state parameter acquired at step S62, and judges whether a comparison result exceeds a predetermined threshold (S63). If the threshold is not exceeded in the judgment, the process immediately ends. If the threshold is exceeded, as shown in Figure 9(C), a message prompting the operator to perform the alignment process again is displayed on the GUI provided on the image displaying section 4 or the operation section 15, and the process is ended.

A specific example of judging whether the alignment process is appropriate or not on the basis of the magnetic field state parameter of the example 4 is illustrated in Figure 9. In the alignment result memory 12, a magnetic field state parameter Pi at that time is stored in a table in association with an alignment number i (consecutive number) which is an identification number of the alignment process, as shown in Figure 9(A). That is, Figure 9(B) shows that alignment is performed in different areas 1, 2 and 3, and alignment numbers 1, 2 and 3 are given in association with the respective areas. Then, if the magnetic field of an area P' is bad when the ultrasound probe 1 is moved into the area P', the message prompting the operator to perform the alignment process again is displayed. For example, even in a case where a stray magnetic field and the like occur when the ultrasound probe 1 exists in the area 1 in Figure 9(B), the message prompting the operator to perform the alignment process again is displayed if magnetic field parameter comparison results for the areas 2 and 3 exceed a threshold.

According to the example 4, if a magnetic field formed by the magnetic sensor unit 7 is disordered, the message prompting the operator to perform an alignment process again is displayed. Therefore, by deleting an alignment result of alignment performed when the magnetic field is disordered from the alignment result memory 12, it is possible to perform an appropriate alignment process.

### Example 5

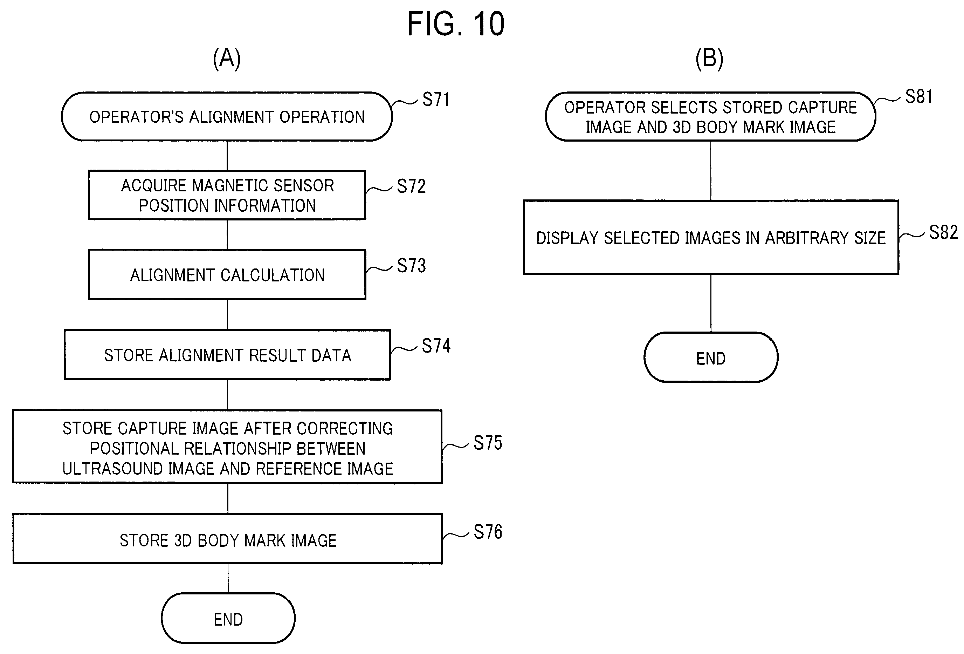

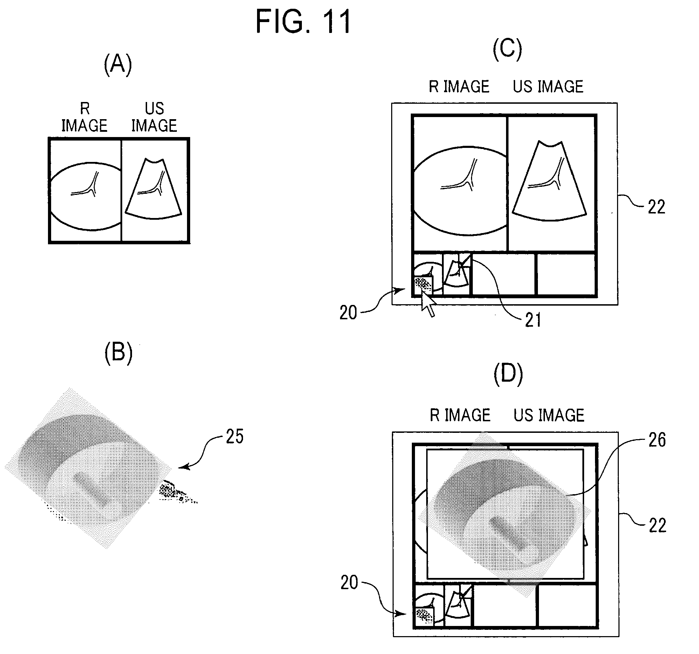

In Figure 10, a process procedure of the image aligning section 10 of an example 5 is shown as a flowchart. Since steps S71 to S75 in Figure 10(A) are the same as S1 to S5 of the example 1, description thereof will be omitted. The present example is characterized in the process of step S76. That is, in the present example, a three-dimensional (3D) body mark image is generated on the basis of volume data of a reference image and displayed on the image displaying section 4 though it is not shown. Especially, a 3D body mark image in which a sectional position of a reference image after being aligned is displayed is stored into the alignment result memory 12. A capture image, which is an alignment result, is shown in Figure 11(A), and a simulated image 25 showing the ultrasound probe 1 and a sectional position on a 3D body mark image is shown in Figure 11(B). Further, as shown in Figure 11(C), thumbnail images 20 each of which includes a capture image and a 3D body mark image are displayed as a list.

Since such a configuration is made, the selected image 25 is displayed being enlarged to an arbitrary size (S82) as shown in Figure 11(D) by selecting a capture image and a 3D body mark image displayed on the image displaying section 4 (S81) as shown in Figure 10(B).

### Reference Signs List

- 1: Ultrasound probe
- 2: Ultrasound image reconstructing section
- 3: Image combining section
- 4: Image displaying section
- 5: Reference image volume data memory
- 6: Reference image reconstructing section
- 7: Magnetic sensor unit
- 10: Image aligning section
- 11: Alignment processing section
- 12: Alignment result memory
- 13: Capture image generating section
- 14: Alignment process selecting section
- 15: Operation section

## Claims

1. An image alignment display method comprising:
performing a process for alignment between an ultrasound image generated on the basis of a reflected echo signal of a cross-section plane of a diagnosing object received with an ultrasound probe and a reference image obtained by another image diagnostic apparatus to display the images on an image displaying section;
storing a plurality of alignment results of the alignment process together with alignment data and correspondence-for-alignment images; and
performing the alignment process by the alignment data corresponding to the stored correspondence-for-alignment images.

2. The image alignment display method according to claim 1, comprising:
displaying the stored correspondence-for-alignment images on the image displaying section as a list; and
when one of the displayed correspondence-for-alignment images is selected, performing the alignment process by the alignment data corresponding to the selected correspondence-for-alignment image.

3. The image alignment display method according to claim 1, wherein the alignment data includes parameter-adjusted data obtained by adjusting a parameter of a preset coordinate conversion formula for bidirectionally converting coordinate data of the ultrasound image and the reference image to perform the alignment process.

4. The image alignment display method according to claim 1, wherein
the alignment data includes detected values of a position and inclination angle of the ultrasound probe detected by a magnetic sensor; and
as for the alignment process, the alignment process is performed by the alignment data corresponding to at least one of the detected values of the position and inclination angle of the ultrasound probe.

5. An ultrasonic diagnostic apparatus comprising:
an ultrasound image reconstructing section configured to generate an ultrasound image on the basis of a reflected echo signal of a cross-section plane of a diagnosing object received by an ultrasound probe;
a volume data memory configured to store volume data of a reference image obtained by another image diagnostic apparatus;
an alignment processing section configured to determine coordinate data of the reference image corresponding to the ultrasound image on the basis of alignment data;
a reference image reconstructing section configured to read out reference image data corresponding to the coordinate data determined by the alignment processing section from the volume data memory to generate a reference image; and
an image displaying section configured to display the ultrasound image and the reference image; wherein
the ultrasonic diagnostic apparatus comprises an alignment result memory configured to store a plurality of alignment results of the alignment processing section together with the alignment data and correspondence-for-alignment images, and
the alignment processing section performs the alignment process by the alignment data corresponding to the correspondence-for-alignment image stored in the alignment result memory.

6. The ultrasonic diagnostic apparatus according to claim 5, comprising:
a correspondence-for-alignment image generating section configured to display the correspondence-for-alignment images stored in the alignment result memory on the image displaying section as a list; and
an alignment process selecting section configured to select one of the correspondence-for-alignment images displayed as the list, wherein
the alignment processing section performs the alignment process by the alignment data corresponding to the correspondence-for-alignment image selected by the alignment process selecting section.

7. The ultrasonic diagnostic apparatus according to claim 5, wherein the alignment data includes parameter-adjusted data obtained by adjusting a parameter of a preset coordinate conversion formula for bidirectionally converting coordinate data of the ultrasound image and the reference image to perform the alignment process.

8. The ultrasonic diagnostic apparatus according to claim 5, wherein
the alignment data includes detected values of a position and inclination angle of the ultrasound probe detected by a magnetic sensor; and
the alignment process selecting section selects the alignment data stored in the alignment result memory on the basis of at least one of the detected values of the position and inclination angle of the ultrasound probe.

9. The ultrasonic diagnostic apparatus according to claim 5, wherein the alignment process selecting section compares at least one of the detected values of the position and inclination angle of the ultrasound probe with a corresponding detected value of the alignment data stored in the alignment result memory and selects the alignment data corresponding to a detected value with a small difference.

10. The ultrasonic diagnostic apparatus according to claim 5, wherein
the alignment data includes the kind of that other image diagnostic apparatus and an identification number of the reference image volume data, and
the alignment process selecting section extracts and selects the alignment data stored in the alignment result memory to which the kind of that other image diagnostic apparatus and the identification number of the reference image volume data correspond.

11. The ultrasonic diagnostic apparatus according to claim 5, wherein the alignment processing section is provided with a function of displaying used alignment data on the image displaying section.

12. The ultrasonic diagnostic apparatus according to claim 5, wherein, together with the correspondence-for-alignment images, a three-dimensional body mark image in which a cross-section plane of the correspondence-for-alignment images is depicted is stored in the alignment result memory.
